# EUROPÄISCHE PATENTSCHRIFT

(11) **EP 1 419 118 B1**
(45) Veröffentlichungstag und Bekanntmachung des Hinweises auf die Patenterteilung: **12.07.2006**
(21) Anmeldenummer: 02772168.7
(22) Anmeldetag: 17.08.2002
(51) Int. Cl.: C03C 3/076, C03C 3/095, C03C 12/00, C03C 4/00, C03C 3/11, C03C 3/062

(54) **ANTIMIKROBIELLES, ENTZÜNDUNGSHEMMENDES, WUNDHEILENDES GLASPULVER UND DESSEN VERWENDUNG**
ANTIMICROBIAL, ANTI-INFLAMMATORY, WOUND-HEALING GLASS POWDER AND USE THEREOF
POUDRE DE VERRE ANTIMICROBIENNE, ANTI-INFLAMMATOIRE, CICATRISANTE ET UTILISATION

(30) Priorität: 22.08.2001 DE 10141116; 20.11.2001 DE 10156577; 27.03.2002 DE 10213630; 27.03.2002 DE 10213632
(43) Veröffentlichungstag der Anmeldung: 19.05.2004
(73) Patentinhaber: Schott AG, 55122 Mainz (DE)
(72) Erfinder: FECHNER, Jörg, Hinrich, 55118 Mainz (DE); ZIMMER, José, 55218 Ingelheim (DE)
(74) Vertreter: Sawodny, Michael-Wolfgang
(86) Internationale Anmeldenummer: PCT/EP2002/009220
(87) Internationale Veröffentlichungsnummer: WO 2003/018496

(56) Entgegenhaltungen:
- EP-A- 0 425 927
- EP-A- 0 921 105
- WO-A-00/76486
- WO-A-96/21628
- JP-A- 7 025 635
- PATENT ABSTRACTS OF JAPAN vol. 1999, no. 13, 30. November 1999 (1999-11-30) & JP 11 228173 A (NIPPON ELECTRIC GLASS CO LTD), 24. August 1999 (1999-08-24)
- PATENT ABSTRACTS OF JAPAN vol. 015, no. 369 (C-0868), 18. September 1991 (1991-09-18) & JP 03 146436 A (U H I SYST KK), 21. Juni 1991 (1991-06-21)
- PATENT ABSTRACTS OF JAPAN vol. 1999, no. 13, 30. November 1999 (1999-11-30) & JP 11 209143 A (ISHIZUKA GLASS CO LTD), 3. August 1999 (1999-08-03)

## Beschreibung

Die Erfindung betrifft ein antimikrobielles, entzündungshemmendes, wundheilendes Glaspulver.

Entzündungshemmende wundheilende Gläser sind aus nachfolgenden Schriften bekannt geworden:
US 5,834,008
WO 00/15167
WO 00/66086

Die entzündungshemmenden und wundheilenden Gläser oder Glaspulver aus derartigen Gläsern umfassen gemäß dem Stand der Technik sämtlich bioaktive Gläser.

Bei den aus den obengenannten Schriften bekannten bioaktiven Gläsern mit entzündungshemmender Wirkung weist das bioaktive Glas einen signifikanten Phosphoranteil > 1 Gew.-% auf.

Die wesentlichen Eigenschaften von bioaktivem Glas sind dem Fachmann bekannt und beispielsweise in der US-A 5,074,916 beschrieben. Danach unterscheidet sich bioaktives Glas von herkömmlichen Kalk-Natrium-Silicat-Gläsern dadurch, daß es lebendes Gewebe bindet.

Bioaktives Glas bezeichnet ein Glas, das eine feste Bindung mit Körpergewebe eingeht, wobei eine Hydroxyl-Apatitschicht ausgebildet wird.

Nachteilig an den bioaktiven Gläsern ist der hohe Phosphoranteil, der beim Erschmelzen der Gläser zu Fertigungsproblemen führt.

Des weiteren sind die bioaktiven Gläser aufgrund ihrer niedrigen hydrolytischen Beständigkeit nur sehr beschränkt für eine Mahlung in wäßrigen Medien geeignet.

Aus der EP 0 425 927 sind Keramiken und glasige Vorprodukte bekannt.

Die Aufgabe der Erfindung ist somit, ein entzündungshemmend und wundheilend wirkendes Glaspulver bereitzustellen, das gegenüber den bioaktiven Gläsern oder Glaspulvern aus derartigen Gläsern einfacher herzustellen, toxikologisch unbedenklich sowie umweltfreundlich ist.

Unter einem Glaspulver wird ein Pulver umfassend eine Vielzahl von Glaspartikeln beliebiger Form, worunter auch Glasfasern fallen verstanden, beispielsweise eine Glaskugel mit Partikelgröße < 1 mm bevorzugt < 500 µm oder eine Glasfaser mit einem Durchmesser von <1 mm bevorzugt < 500 µm.

Des weiteren sollen die Glaspulver Glaszusammensetzung umfassen, die gegenüber Hautreizungen, Hautirritationen, akuten sowie chronischen Wunden bzw. Entzündungen eine hautberuhigende, entzündungshemmende und wundheilende Wirkung zeigen, im Kontakt mit dem Menschen jedoch toxikologisch unbedenklich und insbesondere auch zum Verzehr geeignet sein.

Erfindungsgemäß wird die Aufgabe durch ein entzündungshemmend und wundheilend wirkendes Glaspulver gemäß einem der Ansprüche 1, 4 oder 7 gelöst. Bevorzugt kann das Glas des Glaspulvers 20 - 80 Gew.-% SiO₂, 0 - 40 Gew.-% Na₂O, 0 - 40 Gew.% K₂O, 0 - 40 Gew.% Li₂O, 0 - 40 Gew.-% CaO, 0 - 40 Gew.-% MgO, 0 - 40 Gew.-% Al₂O₃, 0 Gew.-% P₂O₅, 0 - 40 Gew.-% B₂O₃ umfassen. Bevorzugt kann das Glas auch 0 - 30 Gew.-% XFy, wobei X Li, Na, K, Be, Mg, Ca sein kann und y = 1 oder y = 2 ist, enthalten, und/oder 0 - 10 Gew.-% XJ_{y}, wobei X Li, Na, K, Be, Mg, Ca sein kann und y = 1 oder y = 2 ist. Bevorzugt beträgt die Summe Na₂O + K₂O + Li₂O + CaO + MgO 15 bis 80 Gew.-%. Besonders bevorzugt beträgt die Summe Na₂O + K₂O + Li₂O 5 bis 50 Gew.-%. Alternativ hierzu kann die Summe + CaO + MgO 5 bis 50 Gew.-% betragen.

Das erfindungsgemäße Glaspulver kann in großtechnischen Maßstab mit Standardverfahren hergestellt werden, da die Glaszusammensetzung im großtechnischen Maßstab erschmolzen werden kann.

Die Glaspulver gemäß der Erfindung können Produkten zugesetzt werden. Aufgrund ihrer hydrolytischen Beständigkeit können die Glaszusammensetzungen auch in wäßrigen Mahlmedien zu Glaspulvern gemahlen werden.

Zusätzlich zur entzündungshemmenden Wirkung weisen die Glaspulver auch noch antimikrobielle Eigenschaften auf.

Die Gläser, die zu Glaspulvern gemahlen werden, können sehr rein hergestellt werden. Die Glaspulver sind dann toxikologisch unbedenklich sowie zum Verzehr geeignet. Die Belastung durch Schwermetalle ist gering. So beträgt die Maximalkonzentration im Bereich kosmetischer Produkte vorzugsweise für Pb < 20 ppm, Cd < 5 ppm, As < 5 ppm, Sb < 10 ppm, Hg < 1 ppm, Ni < 10 ppm.

Durch die zusätzliche antimikrobielle Wirkung kann eine Konservierung von Produkten selbst erzielt oder eine antimikrobielle Wirkung nach außen erreicht werden. Anwendungsbereiche hierfür sind zum Beispiel kosmetische Produkte, Deoprodukte, Lebensmittel, Farben, Lacke, Putze, Papierhygieneprodukte, Medizinprodukte und Reiniger.

Für bestimmte Anwendungsfelder sind bewußt zugesetzte Mengen an Schwermetallen wie zum Beispiel Ag, Cu, Zn zur Erzielung eines synergistisch verstärkten antimikrobiellen und entzündungshemmenden Effektes vorteilhaft.

Im kosmetischen Bereich spielen Hautirritationen eine wesentliche Rolle. Daher ist es vorteilhaft, wenn das entzündungshemmende und wundheilende Glaspulver besonders hautfreundlich ist.

Ein besonderer Vorteil des erfindungsgemäßen Glaspulvers ist dass es ein Glas umfasst, das aufgrund des Schmelz- und Heißformgebungsverhaltens geeignet ist, um in entsprechenden großtechnischen Anlagen hergestellt zu werden.

Da die Prozeßtemperaturen bzw. die Viskosität des Glases niedrig ist, können kostengünstige Werkstoffe bei Schmelze und Heißformgebung eingesetzt werden.

Neben der Herstellung über ein Schmelzverfahren sind auch alternative Herstellungsverfahren über die Sol-Gel- oder Reaktionssinter-Route denkbar.

Überraschenderweise ergibt sich bei den erfindungsgemäßen Glaspulvern umfassend die angegebenen Glaszusammensetzung eine entzündungshemmende und wundheilende Wirkung die extrem stark sein kann. Je geringer die mittlere Partikelgröße des Glaspulvers, desto höher ist die entzündungshemmende und wundheilende Wirkung wegen der Erhöhung der reaktiven Oberfläche des Glases.

Überraschenderweise wird diese entzündungshemmende und wundheilende Eigenschaft auch bei Glaspulvern umfassend Gläser gefunden, die als Halbzeug eine relativ hohe hydrolytische Beständigkeit besitzen. Mit geringer Partikelgröße und mit einer großen Oberfläche zeigt sich jedoch eine drastische Reaktivitätserhöhung, woraus durch den nachfolgend beschriebenen lonenaustausch entzündungshemmende und wundheilende Wirkung auftritt.

Bei den erfindungsgemäßen Glaspulvern werden durch Reaktionen an der Oberfläche des Glases Alkalien des Glases durch H⁺-lonen des wäßrigen Mediums ausgetauscht. Die entzündungshemmende und wundheilende Wirkung beruht unter anderem auf einer Freisetzung von Ionen sowie auf Oberflächeneffekten der Partikel wie z. B. der Oberflächenladung sowie der antimikrobiellen Wirkung der Glaspulver auf Keime.

Glaspulver mit ionenaustauschbaren Gläsern gemäß der Erfindung wirken in wäßrigen Medien entzündungshemmend und wundheilend durch Ionenaustausch zwischen einem Metallion, wie beispielsweise einem Alkali- oder Erdalkalimetallion und den H⁺-lonen der wäßrigen Lösung sowie durch ionenbedingte Beeinträchtigung des Zellwachstums (osmotischer Druck, Beeinflussung von Stoffwechselvorgängen der Zellen).Die gemahlene Glaspulver mit Teilchen geringer Partikelgröße und großer Oberfläche zeigen eine drastische Reaktivitätserhöhung, woraus, durch den schon beschriebenen lonenaustausch, eine starke entzündungshemmende und wundheilende Wirkung resultiert. Grund hierfür ist der Austausch von Ionen, die eine entzündungshemmende Wirkung besitzen können, bspw. von Kalium. Die entzündungshemmende Wirkung der Glaspulver ist für den Fachmann überraschend, da die Glaszusammensetzungen im ungemahlenen Zustand weitgehend inert sind. Durch das Aufmahlen einer derartigen weitgehend inerten Zusammensetzung kann man erreichen dass im aufgemahlenen Glaspulver lonen wie bspw. K freigesetzt werden, die eine entzündungshemmende Wirkung auf die Epidermis haben. Derartige entzündungshemmenden Eigenschaften hat man im Stand der Technik stets Pulvern aus reaktiven Gläsern mit einem Phosphoranteil größer 1 Gew-% zugeschrieben, da man für die bioaktive und entzündshemmende Wirkung stets die Ausbildung von Hydroxl-Apatit-Schichten als notwendig angesehen hat.

Mit Hilfe von Mahlprozessen können die Glaszusammensetzungen zu Glaspulver mit Partikelgrößen < 100 µm gemahlen werden. Als zweckmäßig haben sich Partikelgrößen < 50 µm bzw. 20 µm erwiesen. Besonders geeignet sind Partikelgrößen < 10 µm sowie kleiner 5 µm. Als ganz besonders geeignet haben sich Partikelgrößen < 2 µm herausgestellt.

Der Mahlprozeß kann sowohl trocken als auch mit wäßrigen und nichtwäßrigen Mahlmedien durchgeführt werden.

Mischungen verschiedener Glaspulver aus dem Zusammensetzungsbereich mit unterschiedlichen Zusammensetzungen und Komgrößen sind möglich, um bestimmte Effekte zu kombinieren.

Je nach Partikelgröße, Konzentration und der Zusammensetzung des Pulvers werden pH-Werte von bis zu 13 erreicht.

Mischungen von Glaspulvern mit unterschiedlichen Zusammensetzungen und Korngrößen können zur Einstellung spezieller Eigenschaften der einzelnen Glaspulver synergistisch kombiniert werden. So ist es beispielsweise möglich, die entzündungshemmende und wundheilende Wirkung des Glaspulvers durch die Partikelgröße zu steuern.

Das Glas des Glaspulvers enthält SiO₂ als Netzwerkbildner, bevorzugt zwischen 35 bis 80 Gew.-%. Bei niedrigen Konzentrationen nimmt die hydrolytische Beständigkeit stark ab, so daß das Mahlen in wäßrigen Medien nicht mehr ohne signifikante Auflösung des Glases gewährleistet ist.

Na₂O wird als Flußmittel beim Schmelzen des Glases eingesetzt. Bei Konzentrationen kleiner 5 % wird das Schmelzverhalten negativ beeinflußt. Außerdem wirkt der notwendige Mechanismus des lonenaustausches nicht mehr hinreichend, um eine entzündungshemmende und wundheilende Wirkung zu erzielen. Bei höheren Na₂O-Konzentrationen als 30 Gew.-% ist eine Verschlechterung der chemischen Resistenz bzw. hydrolytischen Beständigkeit, insbesondere in Verbindung einer Abnahme des SiO₂-Anteils, zu beobachten.

Alkali- und Erdalkalioxide können insbesondere hinzugesetzt werden, um den lonenaustausch zu erhöhen und so die entzündungshemmende und wundheilende Wirkung zu verstärken.

Die Menge an Al₂O₃ kann zur Erhöhung der chemischen Beständigkeit der Kristallisationsstabilität sowie der Steuerung der antimikrobiellen Wirkung bis zu maximal 8 Gew.-% hinzugegeben werden.

B₂O₃ wirkt als Netzwerkbildner und kann auch der Steuerung der entzündungshemmenden und wundheilenden Wirkung dienen.

ZnO ist eine wesentliche Komponente für die Heißformgebungseigenschaften des Glases. Es verbessert die Kristallisationsstabilität und erhöht die Oberflächenspannung. Außerdem kann es den entzündungshemmenden und wundheilenden Effekt unterstützen. Bei geringen SiO₂-Gehalten erhöht es die Kristallisations-stabilität. Zur Erzielung einer entzündungshemmenden und wundheilenden Wirkung können bis zu 8 Gew.-% ZnO enthalten sein. Eine bevorzugte Ausführung enthält < 4 Gew.-% ZnO oder < 2 Gew.-%. Ausführungen mit < 1 Gew.-% oder 0,5 Gew.-% bzw. < 0,1 Gew.-% sind besonders bevorzugt.

Um die entzündungshemmende und wundheilende Wirkung des Grundglases zu verstärken können AgO, CuO als wirkende Zusätze zugegeben werden.

Das erfindungsgemäße Glas ruft keine hautirritierenden Wirkungen hervor.

Durch eine Kombination der pH-Wirkung und der Ag, Cu oder Zn-Abgabe kann eine erheblichen Steigerung der entzündungshemmenden und wundheilenden Wirkung sowie der zusätzlichen antimikrobiellen Wirkung erzielt werden, die über die Summe der Einzelwirkungen deutlich hinausgeht. Die in das Produkt freigesetzte Konzentration von Ag, Cu, Zn-lonen kann hierbei deutlich unter 1 ppm liegen.

Die Einbringung des Ag, Cu, Zn kann hierbei entweder bereits bei der Schmelze durch entsprechende Salze erfolgen oder aber durch lonenaustausch des Glases nach der Schmelze.

Zur Erzielung von Farbwirkungen können den Gläsern einzelne oder auch mehrere farbgebende Komponenten wie z.B. Fe₂O₃, CoO, CuO, V₂O₅, Cr₂O₅ in einer Gesamtkonzentration kleiner 4 Gew.-%, vorzugsweise kleiner 1 Gew.-% zugesetzt werden.

Glaspulver mit innerhalb des beanspruchten Zusammensetzungsbereiches liegenden Gläser erfüllen alle Anforderungen bezüglich eines Einsatzes in den Bereichen Papierhygiene, Kosmetik, Farben, Lacken, Putzen, Medizinprodukten, kosmetischen Anwendungen, Nahrungsmittelzusatz sowie Verwendung in Deoprodukten, Anti-Transpiranten sowie in Produkten zur Behandlung von Hautirritationen, akuten und chronischen Wunden.

Eine Eigenschaft des Glaspulvers, ist die überraschenderweise nachgewiesene Hautverträglichkeit, die auch bei hohen Konzentrationen mit hohen pH-Werten gegeben ist.

Das Glaspulver kann in jeder geeigneten Form eingesetzt werden. Mischungen unterschiedlicher Glaspulver aus dem Zusammensetzungsbereich mit unterschiedlichen Zusammensetzungen sind ebenfalls möglich. Die Mischung mit anderen Glaspulvern ist ebenfalls möglich, um bestimmte Effekte zu kombinieren.

Komponenten wie Fluor können je nach Anwendungsgebiet dem Glas bis zu Konzentrationen von in Summe 5 Gew.-% zugesetzt werden.

Das in dieser Erfindung beschriebene Glas aus dem das erfindungsgemäße Glaspulver durch Mahlen erhalten wird, ist nicht oder schwer wasserlöslich. Das Glaspulver wirkt wirkt in erster Linie durch lonenaustausch bzw. lonenabgabe, was mit einer Oberflächenreaktion, pH-Erhöhung und Metallionen-Freisetzung verbunden ist.

Überraschenderweise zeigen die Glaspulver gemäß der Erfindung einen höheren entzündungshemmenden und wundheilenden Effekt als die Gruppe der bioaktiven Gläser, die im Stand der Technik beschrieben wurden, oder Glaspulvern, die aus derartigen Gläsern hergestellt wurden.

Die Erfindung soll nachfolgend anhand der Ausführungsbeispiele und Figuren beschrieben werden.

Es zeigen:

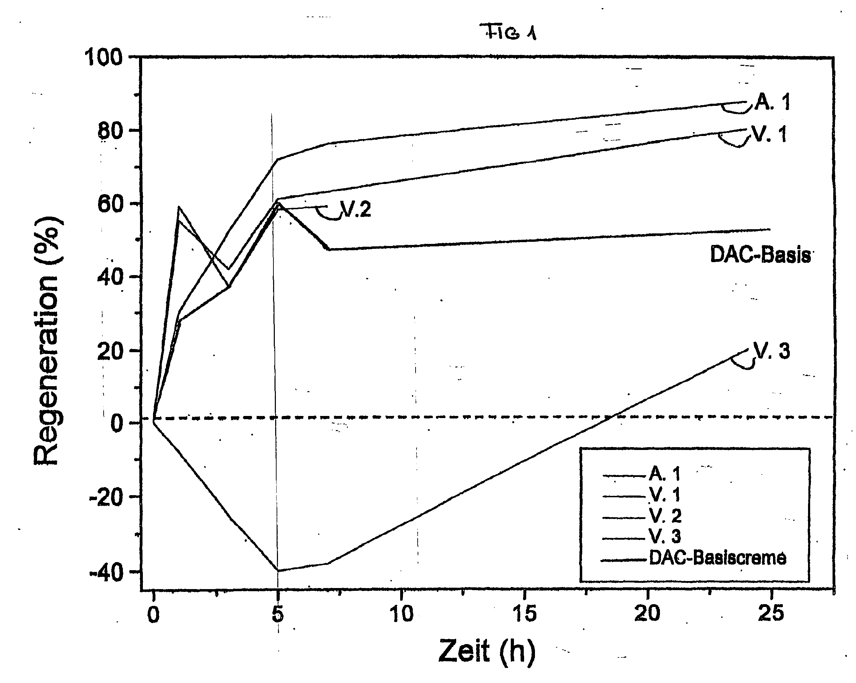

- Figur 1: die Regeneration der Barrierefunktion der Epidermis nach Vorschädigung.

### Ausführungsbeispiele

Aus den Rohstoffen wurde das Glas in einem Kieselglas-Tiegel erschmolzen, das anschließend zu Ribbons verarbeitet wurde. Die Ribbons wurden mittels Trockenmahlung zu Pulver mit einer Partikelgröße d50 = 4 µm weiterverarbeitet.

In Tabelle 2 werden die Zusammensetzungen und Eigenschaften von Gläsern angegeben, die zu den erfindungsgemäßen Glaspulvern gemahlen werden können. Die Zusammensetzungen beziehen sich auf Synthesewerte in Gew.-% auf Oxidbasis.

**Tabelle 1:**

| | V.1 | V.2 | V.3 |
|---|---|---|---|
| SiO₂ | 45,0 | 58,2 | 35,0 |
| Al₂O₃ | | | |
| CaO | 24,5 | 32,6 | 29,5 |
| MgO | | | |
| Fe₂O₃ | | | |
| Na₂O | 24,5 | | 29,5 |
| K₂O | | | |
| P₂O₅ | 6,0 | 9,2 | 6,0 |

**Tabelle 2:**

| | **Ausführungsbeispiel** 1 | **Ausführungsbeispiel 2** | **Ausführungsbeispiel 3** | **Ausführungsbeispiel 4** | **Ausführungsbeispiel 5** | **Ausführungsbeispiel 6** | **Ausführungsbeispiel 7** |
|---|---|---|---|---|---|---|---|
| SiO₂ | 71,2 | 45,0 | 72,0 | 60,0 | 48,0 | 45,0 | 45,0 |
| B₂O₃ | | | | | | | 10,0 |
| Al₂O₃ | 0,35 | | | | | | |
| P₂O₅ | | | | | | 1,0 | |
| CaO | 9,9 | 24,5 | 9,6 | 20,0 | 20,0 | 27,5 | 25,0 |
| MgO | 4,2 | | 4,0 | | 10,0 | | |
| Fe₂O₃ | 0,1 | | | | | | |
| Li₂O | | | | | 2,0 | | |
| Na₂O | 14,2 | 30,5 | 14,4 | 20,0 | 10,0 | 27,5 | 15,0 |
| K₂O | 0,05 | | | | 10,0 | | 5,0 |

In Tabelle 1 sind Vergleichsbeispiele bioaktiver Gläser V1, V2 sowie V3 gezeigt. Tabelle 2 zeigt Ausführungsbeispiele A1 bis A7.

Besonders einfach herzustellen und großtechnisch verfügbar, da in großtechnischen Anlagen erschmelzbar sind Gläser, die die nachfolgenden Komponenten umfassen, wobei die Angaben in Gew-% auf Oxidbasis bezogen sind:

SiO₂: 68 - 75 Gew-%, Na₂O: 10 - 20 Gew-%, Al₂O₃: 0-3 Gew-%, CaO: 5-15 Gew-%, MgO: 0-10 Gew-%. Derartige Gläser zeigen nach dem Aufmahlen eine entzündungshemmende und wundheilende Wirkung. Dies ist für den Fachmann überraschend, da Gläser mit einem Phosphoranteil kleiner 1 Gew-% bezogen auf Oxidbasis, beispielsweise Kalk-Natron-Gläser als Glas weitgehend inreaktiv sind. Eine entzündungshemmende Wirkung wurde lediglich phosphorhaltigen Gläsern mit einem Phosphoranteil > 1 Gew-% zugeschrieben, da diese Gläser als sehr reaktiv gelten.

Alle Gläser, insbesondere die geeigneten Gläser in oben angegebener Zusammensetzung können Spurenelemente und/oder übliche Läutermittel in üblichen Mengen aufweisen. Spurenelemente sind beispielsweise im Glas enthaltene Verunreinigungen wie Fe₂O₃ oder K₂O; übliche Läutermittel As₂O₃, Sb₂O₃.
Die den bioaktiven Gläsern überlegene entzündungshemmende und wundheilende Wirkung zeigen Studien an Mäusen. Es wurden Versuche mit je einer Maus an drei Meßpunkten durchgeführt. Die als Glaspulver untersuchten Wirksubstanzen A.1, V.1, V.2 und V.3 wurden einer Menge von 10 Gew.-% in einer DAC-Basiscreme appliziert. Zum Vergleich mit den mit einer Wirksubstanz versehenen DAC-Creme wurde auch eine DAC-Basiscreme ohne Wirkstoff appliziert.

In Figur 1 ist der zeitliche Verlauf der Reparatur einer oberflächlichen Wunde, d.h. die Regeneration der Barrierefunktion der Epidermis, gemessen über den transepidermalen Wasserverlust, gezeigt. Die oberflächliche Wunde wurde durch eine Vorschädigung mit Tape-Stripping erhalten. Unter Tape-Stripping versteht man die Schädigung der obersten Hautschichten durch das Aufbringen und Abziehen von Klebestreifen.

In Figur 1 ist erkennbar, daß die Regeneration der Barrierefunktion der Haut nach 24 Stunden bei einem Glaspulver mit erfindungsgemäßer Glaszusammensetzung ca. 10 % besser ist als mit dem bioaktiven Glaspulver V.1. Die bioaktiven Glaspulver V.2 und V.3 sowie die DAC-Creme ohne Wirkstoff als Vergleichsproben zeigen eine noch deutlich geringere Regeneration der Barrierefunktion. Die Vergleichsversuche zeigen deutlich die den bioaktiven Glaspulvern überlegene entzündungshemmende, wundheilende Wirkung der erfindungsgemäßen Glaspulver mit den angegebenen Glaszusammensetzung. Des weiteren weisen die Glaspulver gemäß der Erfindung auch eine antimikrobielle Wirkung auf. Diese ist nach Europ. Pharmakopoe (3. Auflage) für das Ausführungsbeispiel A1 mit einer Komgröße d 50 von 4 µm in Tabelle 3 angegeben.

**Tabelle 3:**

| | **E.coli** | **P. aeruginosa** | **S. aureus** | **C. albicans** | **A. niger** |
|---|---|---|---|---|---|
| **Start** | 290000 | 270000 | 250000 | 300000 | 250000 |
| **2 Tage** | 900 | 1800 | 800 | < 100 | 2000 |
| **7 Tage** | < 100 | 200 | < 100 | 0 | 2000 |
| **14 Tage** | 0 | 0 | 0 | 0 | 0 |
| **21 Tage** | 0 | 0 | 0 | 0 | 0 |
| **28 Tage** | 0 | 0 | 0 | 0 | 0 |

Die entzündungshemmend und wundheilend wirkenden Glaspulver können als Nahrungsmittelzusatz, in kosmetischen Produkten und Deoprodukten, insbesondere zur Reduzierung von Hautirritationen, aber nicht beschränkt darauf, in Medizinprodukten, insbesondere Produkten zur Wundversorgung und - behandlung, Kunststoffen und Polymeren, Papierhygiene, in Farben, Lacken sowie in Reinigungsmitteln angewandt werden.

## Patentansprüche

1. Entzündungshemmend und wundheilend wirkendes Glaspulver, wobei das Glas des Glaspulvers die nachfolgenden Komponenten in Gew-% auf Oxidbasis umfaßt:
20 - 80 Gew.-% SiO₂
0 - 40 Gew.-% Na₂O
0 - 40 Gew.-% K₂O
0 - 40 Gew.-% Li₂O
0 - 40 Gew.-% CaO
0 - 40 Gew.-% MgO
0 - 40 Gew.-% Al₂O₃
0 Gew.-% P₂O₅
0 - 40 Gew.-% B₂O₃
0 - 10 Gew.-% ZnO
und gegebenenfalls Spurenelementen und/oder übliche Läutermittel in üblichen Mengen,
wobei die Summe Na₂O + K₂O + Li₂O + CaO + MgO 15 bis 80 Gew.-% beträgt, und das Glas des Glaspulvers frei von Ag ist und die Größe der Glaspartikel des Glaspulvers im Mittel < 100 µm, bevorzugt < 50 µm, besonders bevorzugt < 20 µm ist.

2. Entzündungshemmendes und wundheilend wirkendes Glaspulver, nach Anspruch 1, wobei die Summe Na₂O + K₂O + Li₂O 5 bis 50 Gew-% beträgt.

3. Entzündungshemmendes und wundheilend wirkendes Glaspulver, nach Anspruch 1, wobei die Summe CaO + MgO 5 bis 50 Gew-% beträgt.

4. Entzündungshemmend und wundheilend wirkendes Glaspulver, wobei das Glas des Glaspulvers die nachfolgenden Komponenten in Gew-% auf Oxidbasis umfaßt:
68 - 75 Gew.-% SiO₂
10 - 20 Gew.-% Na₂O
5 - 15 Gew.-% CaO
0 - 10 Gew.-% MgO
0 - 3 Gew.-% Al₂O₃
0 Gew.-% P₂O₅
und gegebenenfalls Spurenelementen und/oder übliche Läutermittel in üblichen Mengen, und die Größe der Glaspartikel des Glaspulvers im Mittel < 100 µm, bevorzugt < 50 µm, besonders bevorzugt < 20 µm ist.

5. Entzündungshemmend und wundheilend wirkendes Glaspulver gemäß einem der Anspruche 1 bis 4, wobei das Glas des Glaspulvers desweiteren 0 - 30 Gew.-% XF_{y}, wobei X Li, Na, K, Be, Mg, Ca sein kann und y=1 oder y=2, umfaßt.

6. Entzündungshemmend und wundheilend wirkendes Glaspulver gemäß einem der Ansprüche 1 bis 5, wobei das Glas des Glaspulvers des weiteren 0 - 10 Gew-% XJ_{y}, wobei X Li, Na, K, Be, Mg, Ca sein kann und y=1 oder y=2 umfaßt.

7. Entzündungshemmend und wundheilend wirkendes Glaspulver, wobei das Glas des Glaspulvers die nachfolgenden Komponenten umfaßt:
20 - 80 Gew.-% SiO₂
0 - 30 Gew.-% Na₂O
0 - 30 Gew.-% K₂O
0 - 30 Gew.-% CaO
0 - 30 Gew.-% MgO
0 - 30 Gew.-% Al₂O₃
0 Gew.-% P₂O₅
0 - 40 Gew.-% B₂O₃
und gegebenenfalls Spurenelemente und/oder übliche Läutermittel in üblichen Mengen und das Glas des Glaspulvers frei von Ag ist und die Größe der Glaspartikel des Glaspulvers im Mittel < 100 µm, bevorzugt < 50 µm, besonders bevorzugt < 20 µm ist.

8. Entzündungshemmend und wundheilend wirkendes Glaspulver nach einem der Ansprüche 1 bis 7 **dadurch gekennzeichnet, daß** das Glas des Glaspulvers desweiteren Schwermetallionen, ausgewählt aus der Klasse der nachfolgenden Elemente:
Cu, Zn
umfaßt

9. Entzündungshemmend und wundheilend wirkendes Glaspulver nach einem der Ansprüche 4 bis 6, **dadurch gekennzeichnet, daß** das Glas des Glaspulvers des weiteren Ag umfasst.

10. Entzündungshemmend und wundheilend wirkendes Glaspulver nach einem der Ansprüche 1 bis 7, **dadurch gekennzeichnet, daß** das Glas des Glaspulvers frei von Schwermetallen ist, insbesondere kein Cu, Zn enthält.

11. Entzündungshemmend und wundheilend wirkendes Glaspulver nach einem der Ansprüche 4 bis 6, **dadurch gekennzeichnet, daß** das Glas des Glaspulvers frei von Ag ist.

12. Entzündungshemmendes und wundheilendes Glaspulver nach einem der Ansprüche 1 bis 11, **dadurch gekennzeichnet, daß** die Größe der Partikel des Glaspulvers im Mittel < 10 µm ist.

13. Entzündungshemmend und wundheilend wirkendes Glaspulver nach einem der Ansprüche 1 bis 11, **dadurch gekennzeichnet, daß** die Größe der Glaspartikel < 5 µm ist.

14. Entzündungshemmend und wundheilend wirkendes Glaspulver nach einem der Ansprüche 1 bis 11, **dadurch gekennzeichnet, daß** die Größe der Glaspartikel des Glaspulvers im Mittel < 1 µm ist.

15. Verwendung eines entzündungshemmend und wundheilend wirkenden Glaspulvers nach einem der Ansprüche 1 bis 14 in Kosmetikprodukten.

16. Verwendung eines entzündungshemmend und wundheilend wirkenden Glaspulvers nach einem der Ansprüche 1 bis 14 in Deodorantprodukten.

17. Verwendung eines entzündungshemmend und wundheilend wirkenden Glaspulvers nach einem der Ansprüche 1 bis 14 in medizinischen Produkten und Präparaten.

18. Verwendung eines entzündungshemmend und wundheilend wirkenden Glaspulvers nach einem der Ansprüche 1 bis 16 in Kunststoffen und Polymeren.

19. Verwendung eines entzündungshemmend und wundheilend wirkenden Glaspulvers nach einem der Ansprüche 1 bis 14 im Bereich der Papierhygiene.

20. Verwendung eines entzündungshemmend und wundheilend wirkenden Glaspulvers nach einem der Ansprüche 1 bis 14 in Nahrungsmitteln.

21. Verwendung eines entzündungshemmend und wundheilend wirkenden Glaspulvers nach einem der Ansprüche 1 bis 14 in Reinigungsmitteln.

22. Verwendung eines entzündungshemmend und wundheilend wirkende Glaspulvers nach einem der Ansprüche 1 bis 14 in Anti-Transpiranten.

23. Verwendung eines entzündungshemmend und wundheilend wirkenden Glaspulvers nach einem der Ansprüche 1 bis 14 in Farben und Lacken.

24. Verwendung eines entzündungshemmend und wundheilend wirkenden Glaspulvers nach einem der Ansprüche 1 bis 14 in Produkten zur . Behandlung von Hautirritationen, akuten und chronischen Wunden.

25. Verfahren zur Herstellung eines entzündungshemmenden und wundheilend wirkenden Glaspulvers nach einem der Ansprüche 1 bis 14 umfassend die folgenden Schritte:
25.1 es wird aus Rohstoffen ein Glas erschmolzen
25.2 das erschmolzene Glas wird zu Ribbons weiterverarbeitet
25.3 die Ribbons werden zu Glaspulver gemahlen, wobei die Größe der Glaspartikel des Glaspulvers im Mittel < 100 µm, bevorzugt < 50 µm, besonders bevorzugt < 20 µm ist

## Claims

1. An anti-inflammatory and wound-healing glass powder, with the glass of the glass powder comprising the following components in weight percent on oxide basis:
20 to 80% by weight of SiO₂
0 to 40% by weight of Na₂O
0 to 40% by weight of K₂O
0 to 40% by weight of Li₂O
0 to 40% by weight of CaO
0 to 40% by weight of MgO
0 to 40% by weight of Al₂O₃
0% by weight of P₂O₅
0 to 40% by weight of B₂O₃
0 to 10% by weight of ZnO
and optionally trace elements and/or usual refining agents in the usual quantities, with the sum total of Na₂O + K₂O + Li₂O + CaO + MgO being 15 to 80% by weight, and the glass of the glass powder being free of Ag and the size of the glass particles of the glass powder being on average < 100 µm, preferably < 50 µm, more preferably < 20 µm.

2. An anti-inflammatory and wound-healing glass powder according to claim 1, with the sum total of Na₂O + K₂O + Li₂O being 5 to 50% by weight.

3. An anti-inflammatory and wound-healing glass powder according to claim 1, with the sum total of CaO + MgO being 5 to 50% by weight.

4. An anti-inflammatory and wound-healing glass powder, with the glass of the glass powder comprising the following components in weight percent on oxide basis:
68 to 75% by weight of SiO₂
10 to 20% by weight of Na₂O
5 to 15% by weight of CaO
0 to 10% by weight of MgO
0 to 3% by weight of Al₂O₃
0% by weight of P₂O₅
and optionally trace elements and/or usual refining agents in the usual quantities, and the size of the glass particles of the glass powder is on average < 100 µm, preferably < 50 µm, more preferably < 20 µm.

5. An anti-inflammatory and wound-healing glass powder according to one of the claims 1 to 4, with the glass of the glass powder further comprising 0 to 30% by weight of XF_{y}, wherein X may be Li, Na, K, Be, Mg, Ca and y=1 or y=2.

6. An anti-inflammatory and wound-healing glass powder according to one of the claims 1 to 5, with the glass of the glass powder further comprising 0 to 10% by weight of XJ_{y}, wherein X may be Li, Na, K, Be, Mg, Ca and y=1 or y=2.

7. An anti-inflammatory and wound-healing glass powder, with the glass of the glass powder comprising the following components:
20 to 80% by weight of Si02
0 to 30% by weight of Na₂O
0 to 30% by weight of K₂O
0 to 30% by weight of CaO
0 to 30% by weight of MgO
0 to 30% by weight of Al₂O₃
0% by weight of P₂O₅
0 to 40% by weight of B₂O₃
and optionally trace elements and/or usual refining agents in the usual quantities, and the glass of the glass powder is free of Ag and the size of the glass particles of the glass powder is on average < 100 µm, preferably < 50 µm, more preferably < 20 µm.

8. An anti-inflammatory and wound-healing glass powder according to one of the claims 1 to 7, **characterized in that** the glass of the glass powder further comprises heavy metal ions, chosen from the class of the following elements:
Cu, Zn.

9. An anti-inflammatory and wound-healing glass powder according to one of the claims 4 to 6, **characterized in that** the glass of the glass powder further comprises Ag.

10. An anti-inflammatory and wound-healing glass powder according to one of the claims 1 to 7, **characterized in that** the glass of the glass powder is free of heavy metals, especially not containing any Cu, Zn.

11. An anti-inflammatory and wound-healing glass powder according to one of the claims 4 to 6, **characterized in that** the glass of the glass powder is free of Ag.

12. An anti-inflammatory and wound-healing glass powder according to one of the claims 1 to 11, **characterized in that** the size of the particles of the glass powders is on average < 10 µm.

13. An anti-inflammatory and wound-healing glass powder according to one of the claims 1 to 11, **characterized in that** the size of the glass particles is < 5 µm.

14. An anti-inflammatory and wound-healing glass powder according to one of the claims 1 to 11, **characterized in that** the size of the glass particles is on average < 1 µm.

15. The use of an anti-inflammatory and wound-healing glass powder according to one of the claims 1 to 14 in cosmetic products.

16. The use of an anti-inflammatory and wound-healing glass powder according to one of the claims 1 to 14 in deodorant products.

17. The use of an anti-inflammatory and wound-healing glass powder according to one of the claims 1 to 14 in medical products and preparations.

18. The use of an anti-inflammatory and wound-healing glass powder according to one of the claims 1 to 16 in plastic material and polymers.

19. The use of an anti-inflammatory and wound-healing glass powder according to one of the claims 1 to 14 in the area of paper hygiene.

20. The use of an anti-inflammatory and wound-healing glass powder according to one of the claims 1 to 14 in foodstuffs.

21. The use of an anti-inflammatory and wound-healing glass powder according to one of the claims 1 to 14 in cleaning materials.

22. The use of an anti-inflammatory and wound-healing glass powder according to one of the claims 1 to 14 in anti-transpirants.

23. The use of an anti-inflammatory and wound-healing glass powder according to one of the claims 1 to 14 in paints and lacquers.

24. The use of an anti-inflammatory and wound-healing glass powder according to one of the claims 1 to 14 in products for treating skin irritations, acute and chronic wounds.

25. A method for producing an anti-inflammatory and wound-healing glass powder according to one of the claims 1 to 14, comprising the following steps:
25.1 a glass is molten from raw materials;
25.2 the molten glass is further processed into ribbons;
25.3 the ribbons are ground into glass powder, with the size of the glass particles of the glass powder being on average < 100 µm, preferably < 50 µm, more preferably < 20 µm.

## Revendications

1. Poudre de verre ayant un effet anti-inflammatoire et cicatrisant, **caractérisée en ce que** le verre de la poudre de verre contient les composants suivants, en pourcentage en poids sur la base de l'oxyde :
20 à 80 % en poids de SiO₂
0 à 40 % en poids de Na₂O
0 à 40 % en poids de K₂O
0 à 40 % en poids de Li₂O
0 à 40 % en poids de CaO
0 à 40 % en poids de MgO
0 à 40 % en poids d'Al₂O₃
0 % en poids de P₂O₅
0 à 40 % en poids de B₂O₃
0 à 10 % en poids de ZnO
et éventuellement des éléments traces et/ou les agents d'affinage habituels dans les quantités habituelles,
et **en ce que** la somme de Na₂O + K₂O + Li₂O + CaO + MgO représente 15 à 80 % en poids et le verre de la poudre de verre ne contient pas d'Ag et la taille des particules de verre de la poudre de verre est en moyenne < 100 µm, de préférence < 50 µm et en particulier < 20 µm.

2. Poudre de verre ayant un effet anti-inflammatoire et cicatrisant selon la revendication 1, **caractérisée en ce que** la somme de Na₂O + K₂O + Li₂O représente 5 à 50 % du poids.

3. Poudre de verre ayant un effet anti-inflammatoire et cicatrisant selon la revendication 1, **caractérisée en ce que** la somme de CaO + MgO représente 5 à 50 % du poids.

4. Poudre de verre ayant un effet anti-inflammatoire et cicatrisant, **caractérisée en ce que** le verre de la poudre de verre contient les composants suivants, en pourcentage en poids sur la base de l'oxyde :
68 à 75 % en poids de SiO₂
10 à 20 % en poids de Na₂O
5 à 15 % en poids de CaO
0 à 10 % en poids de MgO
0 à 3 % en poids d'Al₂O₃
0 % en poids de P₂O₅
et éventuellement des éléments traces et/ou les agents d'affinage habituels dans les quantités habituelles, **caractérisée en ce que** la taille des particules de verre de la poudre de verre est en moyenne < 100 µm, de préférence < 50 µm et en particulier < 20 µm.

5. Poudre de verre ayant un effet anti-inflammatoire et cicatrisant selon l'une quelconque des revendications 1 à 4, **caractérisée en ce que** le verre de la poudre de verre contient en outre 0 à 30 % en poids de XF_{y}, où X peut être Li, Na, K, Be, Mg, Ca et y = 1 ou y = 2.

6. Poudre de verre ayant un effet anti-inflammatoire et cicatrisant selon l'une quelconque des revendications 1 à 5, **caractérisée en ce que** le verre de la poudre de verre contient en outre 0 à 10 % en poids de XJ_{y}, où X peut être Li, Na, K, Be, Mg, Ca et y = 1 ou y = 2.

7. Poudre de verre ayant un effet anti-inflammatoire et cicatrisant, **caractérisée en ce que** le verre de la poudre de verre contient les composants suivants, en pourcentage en poids sur la base de l'oxyde :
20 à 80 % en poids de SiO₂
0 à 30 % en poids de Na₂O
0 à 30 % en poids de K₂O
0 à 30 % en poids de CaO
0 à 30 % en poids de MgO
0 à 30 % en poids d'Al₂O₃
0 % en poids de P₂O₅
0 à 40 % en poids de B₂O₃
et éventuellement des éléments traces et/ou les agents d'affinage habituels dans les quantités habituelles, et **en ce que** le verre de la poudre de verre ne contient pas d'Ag et la taille des particules de verre de la poudre de verre est en moyenne < 100 µm, de préférence < 50 µm et en particulier < 20 µm.

8. Poudre de verre ayant un effet anti-inflammatoire et cicatrisant selon l'une quelconque des revendications 1 à 7, **caractérisée en ce que** le verre de la poudre de verre contient en outre des ions de métaux lourds, sélectionnés dans la classe contenant les éléments : Cu, Zn.

9. Poudre de verre ayant un effet anti-inflammatoire et cicatrisant selon l'une quelconque des revendications 4 à 6, **caractérisée en ce que** le verre de la poudre de verre contient en outre de l'Ag.

10. Poudre de verre ayant un effet anti-inflammatoire et cicatrisant selon l'une quelconque des revendications 4 à 6, **caractérisée en ce que** le verre de la poudre de verre ne contient pas de métaux lourds, et en particulier pas de Cu ni de Zn.

11. Poudre de verre ayant un effet anti-inflammatoire et cicatrisant selon l'une quelconque des revendications 4 à 6, **caractérisée en ce que** le verre de la poudre de verre ne contient pas d'Ag.

12. Poudre de verre ayant un effet anti-inflammatoire et cicatrisant selon l'une quelconque des revendications 1 à 11, **caractérisée en ce que** la taille des particules de la poudre de verre est en moyenne < 10 µm.

13. Poudre de verre ayant un effet anti-inflammatoire et cicatrisant selon l'une quelconque des revendications 1 à 11, **caractérisée en ce que** la taille des particules de la poudre de verre est en moyenne < 5 µm.

14. Poudre de verre ayant un effet anti-inflammatoire et cicatrisant selon l'une quelconque des revendications 1 à 11, **caractérisée en ce que** la taille des particules de la poudre de verre est en moyenne < 1 µm.

15. Utilisation d'une poudre de verre ayant un effet anti-inflammatoire et cicatrisant selon l'une quelconque des revendications 1 à 14 dans des cosmétiques.

16. Utilisation d'une poudre de verre ayant un effet anti-inflammatoire et cicatrisant selon l'une quelconque des revendications 1 à 14 dans des déodorants.

17. Utilisation d'une poudre de verre ayant un effet anti-inflammatoire et cicatrisant selon l'une quelconque des revendications 1 à 14 dans des produits et préparations médicinaux.

18. Utilisation d'une poudre de verre ayant un effet anti-inflammatoire et cicatrisant selon l'une quelconque des revendications 1 à 16 dans des matières plastiques et de polymères.

19. Utilisation d'une poudre de verre ayant un effet anti-inflammatoire et cicatrisant selon l'une quelconque des revendications 1 à 14 dans le domaine de l'hygiène du papier.

20. Utilisation d'une poudre de verre ayant un effet anti-inflammatoire et cicatrisant selon l'une quelconque des revendications 1 à 14 dans des produits alimentaires.

21. Utilisation d'une poudre de verre ayant un effet anti-inflammatoire et cicatrisant selon l'une quelconque des revendications 1 à 14 dans des détergents.

22. Utilisation d'une poudre de verre ayant un effet anti-inflammatoire et cicatrisant selon l'une quelconque des revendications 1 à 14 dans des antitranspirants.

23. Utilisation d'une poudre de verre ayant un effet anti-inflammatoire et cicatrisant selon l'une quelconque des revendications 1 à 14 dans des peintures et des vernis.

24. Utilisation d'une poudre de verre ayant un effet anti-inflammatoire et cicatrisant selon l'une quelconque des revendications 1 à 14 dans des produits pour le traitement des irritations cutanées et des plaies aiguës et chroniques.

25. Procédé de fabrication d'une poudre de verre ayant un effet anti-inflammatoire et cicatrisant selon l'une quelconque des revendications 1 à 14, comprenant les étapes suivantes :
25.1 fusion des matières premières pour obtenir un verre
25.2 traitement du verre fondu pour obtenir des rubans
25.3 broyage des rubans pour obtenir de la poudre de verre, la taille des particules de verre de la poudre de verre étant en moyenne < 100 µm, de préférence < 50 µm et en particulier < 20 µm.
